(19) Europäisches Patentamt
European Patent Office
Office européen des brevets

(11) **EP 3 584 329 A1**

(12) **EUROPEAN PATENT APPLICATION**
published in accordance with Art. 153(4) EPC

(43) Date of publication:
**25.12.2019 Bulletin 2019/52**

(51) Int Cl.:
*C21C 5/46* (2006.01)    *G01N 21/17* (2006.01)
*G01N 33/20* (2019.01)

(21) Application number: **18754452.3**

(22) Date of filing: **13.02.2018**

(86) International application number:
**PCT/JP2018/004818**

(87) International publication number:
**WO 2018/151075 (23.08.2018 Gazette 2018/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **14.02.2017 JP 2017025441**

(71) Applicant: **Nippon Steel Corporation Tokyo 1008071 (JP)**

(72) Inventors:
• **KUSUNOKI Tomoyuki**
**Tokyo 100-8071 (JP)**
• **MIYAZAKI Takahiro**
**Tokyo 100-8071 (JP)**

(74) Representative: **Vossius & Partner Patentanwälte Rechtsanwälte mbB Siebertstrasse 3 81675 München (DE)**

(54) **METHOD FOR DETECTING SLAG IN MOLTEN STEEL FLOW**

(57)    A method of detecting slag in a molten steel flow includes an image capturing step of sequentially capturing a molten steel flow which is directed from a converter toward a ladle and includes molten steel and slag to acquire a plurality of captured images of the molten steel flow, a histogram creation step of creating a histogram for each captured image, a maximum peak point detection step of detecting a maximum peak point, in which the number of pixels is an absolute maximum value, for each histogram, and a maximum peak point type determination step of determining to which of the slag or the molten steel the maximum peak point of each histogram corresponds.

FIG. 1

## Description

[Technical Field of the Invention]

The present invention relates to a method of detecting slag in a molten steel flow.

Priority is claimed on Japanese Patent Application No. 2017-025441, filed February 14, 2017, the content of which is incorporated herein by reference.

[Related Art]

When molten steel is tapped from a converter to a ladle, generally, the converter is tilted to cause a molten steel flow to be directed from the converter toward the ladle. In this case, it is ideal to leave slag in the converter and cause only the molten steel to flow from the converter to the ladle. However, generally, in the molten steel flow directed from the converter toward the ladle, substantially only the molten steel exists in an early stage of the tapping, but the molten steel and the slag exist between a middle stage of the tapping and a last stage of the tapping. Accordingly, when trying to suppress an outflow of the slag, a residual amount of the molten steel in the converter increases, and thus, there is a concern that a yield may become low.

Meanwhile, when trying to reduce the residual amount of the molten steel in the converter, the slag flows out toward the ladle together with the molten steel, and thus, a large amount of slag exists in the ladle. As a result, there are problems that blowout of the slag from the ladle occurs or component deviation of the molten steel occurs in a secondary refining step which is a later step.

Accordingly, it is desirable to detect the slag in the molten steel flow directed from the converter toward the ladle, to quantify an amount of outflow of the slag, and to control the amount of outflow of the slag to a range required in a tapping operation of the converter.

An emissivity of the slag is higher than that of the molten steel, if an image of the molten steel flow is captured, a region in which the slag exists is image-captured brighter than a region in which the slag does not exist and only the molten steel exists. In other words, a density (gray level) of a pixel region corresponding the slag in a captured image obtained by capturing the image of the molten steel flow is larger than a density of a pixel region corresponding to the molten steel. For example, a technology of detecting the slag using this principle, there are methods described in Patent Documents 1 and 2.

Patent Document 1 discloses a method of capturing an image of a molten steel flow (tapped stream), determining, depending on whether or not a pixel region of the captured image is within a range of a density (gray level) set manually in advance, whether a pixel of the pixel region is a pixel corresponding to the molten steel or a pixel corresponding to the slag, and stopping tapping in a case where a value obtained by dividing the number of pixels corresponding to the slag by a sum of the number of pixels corresponding to the slag and the number of pixels corresponding to the molten steel exceeds a predetermined threshold value.

That is, in the method of Patent Document 1, in the captured image, it is determined that the pixel region within the range of the density set manually in advance is the pixel region corresponding to the slag, and thus, the slag in the molten steel flow is detected.

However, for example, a temperature of the molten steel flow is changed to 100°C or more, depending on a kind of steel or a condition of a tapping operation. In this case, in the captured image, the densities of the pixel regions corresponding to the molten steel and the slag are largely changed. However, in the method of Patent Document 1, a fixed value is used as the predetermined threshold value, and thus, in a case where a condition of the tapping operation of the converter or the like changed, it is difficult to accurately detect the slag.

Patent Document 2 discloses a method of creating a density (brightness) histogram in which a density (brightness) of a captured image obtained by capturing an image of a molten steel flow is shown on a horizontal axis and the number of pixels thereof is shown on a vertical axis and detecting slag using this density histogram. Specifically, in the method of Patent Document 2, in the density histogram, a maximum peak point (maximum peak position) having a largest number of pixels is considered to correspond to the molten steel, a pixel having a density value (brightness value) of N1 or more considering a standard deviation $\sigma$ in a horizontal axis direction of the maximum peak point is determined as the molten steel, and a pixel having a density value (brightness value) of N2 or more obtained by adding a bias value B to the density value N1 is determined as the slag.

According to the method described in Patent Document 2, in the captured image, for example, in a case where the densities of the pixel regions corresponding to the molten steel and the slag are largely changed depending a kind of steel, the maximum peak point is also changed, and thus, it is considered that the density value N2 for detecting the pixel region corresponding to the slag according to the change is automatically changed. Therefore, according to the method of Patent Document 2, it is considered the above-described problem generated in the case where the fixed threshold value (density range) is used like the method of Patent Document 1 can be solved to some extent.

However, the present inventors examined, and thus, it turned out that the maximum peak point in the density histogram does not necessarily correspond to the molten steel, but may correspond to the slag. Therefore, it is difficult to accurately detect the slag by the method of Patent Document 2 which determines the density value N2, assuming that the maximum peak point always corresponds to the molten steel.

[Prior Art Document]

[Patent Document]

[Patent Document 1] Japanese Unexamined Patent Application, First Publication No. 2001-107127

[Patent Document 2] Japanese Unexamined Patent Application, First Publication No. 2006-213965

[Disclosure of the Invention]

[Problems to be Solved by the Invention]

The present invention is made in consideration of the above-described circumstances, and an object thereof is to provide a method of detecting slag in a molten steel flow capable of accurately detecting the slag in the molten steel flow in even a case where a condition of a tapping operation or the like in a converter or the like is changed.

[Means for Solving the Problem]

In order to solve the above-mentioned problem, the present inventors intensively studied.

First, the present inventors captured images of various molten steel flows over an early stage of tapping, a middle stage of the tapping, and a last stage of the tapping using an image capturing unit such as a thermal image camera (thermography) having a main sensitivity in an infrared light region, and thereby many captured images were obtained. In addition, for each of the captured images, a histogram was created in which a temperature was shown on a horizontal axis and the number of pixels was shown on a vertical axis. For example, in a temperature range of the horizontal axis of 1000° to 2000°C, the present inventors found that there might be a maximum peak point in which the number of the pixels of the vertical axis was a maximum value, and if the maximum peak point might be positioned on a low temperature side, the maximum peak point might be positioned on a high temperature side.

Next, the present inventors found that, in a case where slag did not exist in the molten steel flow or in a case where a very small amount of slag existed in the molten steel flow (for example, the early stage of the tapping to the middle stage of the tapping), in the histogram, the maximum peak point corresponded to the molten steel and was positioned on a low temperature side, and in a case where a large amount of slag did exist (in a case where the slag did exist in the molten steel flow to an extent in which the slag could be easily observed visually in the last stage of the tapping), the maximum peak point corresponded to the slag and was positioned on a high temperature side. However, as described above, a temperature of the molten steel flow is changed depending on a kind of steel or a condition of a tapping operation. Accordingly, in a case where it is determined to which of the molten steel and the slag the maximum peak point corresponds by determining on which of the low temperature side and the high temperature side of a fixed threshold value the maximum peak point is positioned using the fixed threshold value, it is difficult to accurately detect the slag.

Accordingly, the present inventors more intensively studied, and as a result, the present inventors found that in a case where the slag did not exist in the molten steel flow or a very small amount of slag existed in the molten steel flow, a temperature variation (for example, the temperature variation per 0.2 seconds is less than 100°C) of the maximum peak point was small. Specifically, in general, the molten steel refined by the converter is stirred by top blowing or top bottom blowing in the converter at the time of the refining, and thus, a temperature dispersion is small. Therefore, the molten steel flow directed out from the converter has a small temperature dispersion, for example, a temperature variation less than 100°C per 0.2 seconds.

In addition, since whiteness (brightness) of the slag is higher (emissivity of the slag is about 1.5 times emissivity of the molten steel) than that of the molten steel, the present inventors focused on a phenomenon in which when a temperature of the molten steel was measured by the emissivity of the molten steel, a temperature of the slag was apparently higher than an actual temperature by at least 100°C or more, and in most cases, about several hundreds of °C (for example, 300°C to 600°C) higher than the actual temperature. If this phenomenon is used, for the molten steel and the slag (that is, the molten steel flow which is directed out from the converter and includes the molten steel and the slag) having the approximately same actual temperature, an apparent relative temperature difference between the molten steel and the slag can be made remarkable. Accordingly, the present inventors found that the slag in the molten steel flow could be accurately detected if the apparent relative temperature difference was used.

Based on the above findings, the present invention adopts the following in order to solve the above problems.

(1) According to an aspect of the present invention, there is provided a method of detecting slag in a molten steel flow, including: an image capturing step of sequentially capturing a molten steel flow which is directed from a converter toward a ladle and includes molten steel and slag to acquire a plurality of captured images of the molten steel flow; a histogram creation step of creating a histogram, in which a density parameter corresponding to a density of each pixel constituting each captured image is shown on a horizontal axis and the number of pixels that is a total number of pixels each having the density parameter is shown on a vertical axis, for each captured image; a maximum peak point detection step of detecting a maximum peak point, in which the number of pixels is an absolute maximum value, for each histogram; and a maximum peak point type determination step of determining to which of the slag or the molten steel the maximum peak point of each histogram corresponds, in which when a maximum peak point $P_n$ of a histogram of an nth ($n \geq 2$) captured image is determined in the maximum peak point type determination step, in a case where a variation $\Delta T$ of a density parameter $T_n$ at the maximum peak point $P_n$ with respect to a density parameter $T_{n-1}$ at a maximum peak point $P_{n-1}$ in a histogram of an n-1th captured image is equal to or more than a predetermined value, it is determined that the maximum peak point $P_n$ corresponds to the slag, and in a case where a variation $\Delta T$ is less than the predetermined value, if a variation $\Delta T'$ of the density parameter $T_n$ with respect to a density parameter $T_j$ at a maximum peak point $P_j$ in a histogram of a jth ($j \leq n-1$) captured image, in which a maximum peak point is determined as the molten steel, is equal to or more than the predetermined value, it is determined that the maximum peak point $P_n$ corresponds to the slag, and if a variation $\Delta T'$ is less than the predetermined value, it is determined that the maximum peak point $P_n$ corresponds to the molten steel.

(2) In the aspect described in (1), the maximum peak point type determination step may include, as the maximum peak point $P_j$, using a maximum peak point in a histogram of a captured image which is acquired before the nth captured image and is closest in acquisition order to the nth captured image, and in which a maximum peak point is determined as the molten steel.

(3) In the aspect described in (1) or (2), the method of detecting slag may further include a first determination step of, in a case where it is determined that the maximum peak point corresponds to the slag in the maximum peak point type determination step, determining that a pixel having a density parameter less than a first threshold value determined based on the maximum peak point corresponds to the molten steel and a pixel having a density parameter equal to or more than the first threshold value corresponds to the slag; and a second determination step of, in a case where it is determined that the maximum peak point corresponds to the molten steel in the maximum peak point type determination step, determining that a pixel having a density parameter equal to or less than a second threshold value determined based on the maximum peak point corresponds to the molten steel and a pixel having a density parameter more than the second threshold value corresponds to the slag.

(4) In the aspect described in (3), the first threshold value may be represented by a first straight line, which passes through the maximum peak point and has a positive inclination, in the histogram, the second threshold value may be represented by a second straight line, which passes through the maximum peak point and has a negative inclination, in the histogram, and an absolute value of an inclination of the second straight line may be larger than an absolute value of an inclination of the first straight line.

(5) In the aspect described in (4), the first straight line may be a straight line which passes through a peak point having a maximum density parameter from among points having the number of pixels less than a threshold value of the number of pixels and a small density parameter equal to or more than a predetermined value with respect to the density parameter at the maximum peak point, and the maximum peak point, and the absolute value of the inclination of the second straight line may be 1.5 to 2.5 times the absolute value of the inclination of the first straight line.

[Effects of the Invention]

According to each aspect of the present invention, it is possible to accurately detect slag in a molten steel flow in even a case where a condition of a tapping operation or the like in a converter or the like is changed.

[Brief Description of the Drawings]

FIG. 1 is a schematic view showing a schematic configuration of a slag detection device used in a slag detection method according to an embodiment of the present invention.
FIG. 2 is a flowchart showing a schematic procedure of the slag detection method.
FIG. 3 is a flowchart showing a schematic procedure of a maximum peak point type determination step ST4 shown in FIG. 2.
FIG. 4A is a view showing an example of a captured image acquired in an image capturing step ST1 shown in FIG. 2.
FIG. 4B is a graph showing a histogram created based on the captured image of FIG. 4A in a histogram creation step ST2 shown in FIG. 2.
FIG. 5A is a view showing an example of the captured image acquired in the image capturing step ST1 shown in FIG. 2 and is a view showing an example different from that of FIG. 4A.
FIG. 5B is a graph showing a histogram created based on the captured image of FIG. 5A in the histogram creation step ST2 shown in FIG. 2.
FIG. 6 is a graph for explaining an example of a determination method of a predetermined value Th1.
FIG. 7 is a view for explaining the maximum peak point type determination step ST4 shown in FIG. 2.

FIG. 8 is a graph for explaining a first threshold value determined in a first determination step ST5 shown in FIG. 2.

FIG. 9A is a view showing an example of the captured image acquired in the image capturing step ST1 shown in FIG. 2 and is a view showing an example different from those of FIGS. 4A and 5A.

FIG. 9B is a graph showing a histogram created based on the captured image of FIG. 9A in the histogram creation step ST2 shown in FIG. 2 and is a graph for explaining a second threshold value determined in a second determination step ST6.

FIG. 10A is a graph for explaining a slag detection method described in Patent Document 2.

FIG. 10B is a view showing a captured image used to create the histogram of FIG. 10A.

[Embodiments of the Invention]

Hereinafter, a method of detecting slag (hereinafter, simply referred to as a "slag detection method") in a molten steel flow according to an embodiment of the present invention will be described with reference to the drawings. In addition, in the present specification and drawings, the same reference symbol is assigned to the same component having substantially the same functional configuration, and repeated descriptions thereof are omitted.

First, a configuration of a slag detection device 100 used in the slag detection method according to the present embodiment will be described.

<Configuration of Slag Detection Device 100 of Present Embodiment>

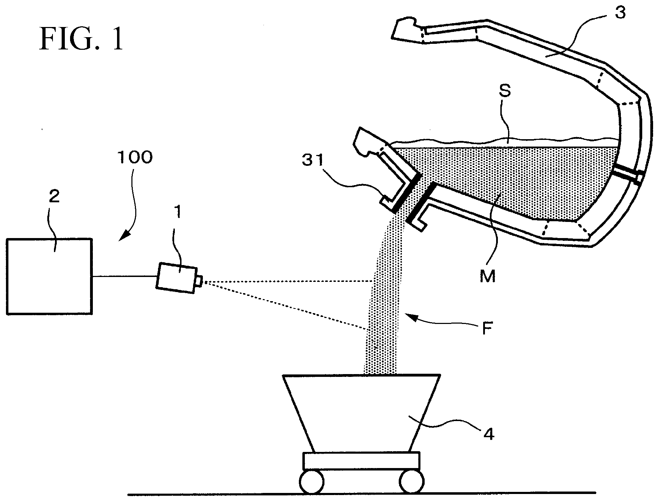

FIG. 1 is a schematic view showing a schematic configuration of the slag detection device 100. In addition, in FIG. 1, a converter 3 which accommodates molten steel M and slag S is shown in cross section.

As shown in FIG. 1, when tapping from the converter 3 to a ladle 4 is performed, the slag detection device 100 is used to detect the slag S in the molten steel flow F directed from a tapping hole 31 of the tilted converter 3 toward the ladle 4.

Here, in a state (that is, a state before the converter 3 is tilted) before the tapping from the converter 3 to the ladle 4 is performed, in the converter 3, the molten steel M and the slag S are separated from each other to some extent (the slag S is positioned on a surface of the molten steel M in the converter 3). Accordingly, if the converter 3 is tilted, first, the molten steel M flows out from the tapping hole 31 provided on a side surface of the converter 3, and the slag S starts to flow out at a certain point thereafter. That is, the molten steel flow F mainly includes the molten steel M in an early stage of the tapping. However, for example, in a middle stage of the tapping to a last stage of the tapping, the molten steel flow F includes the slag S and the molten steel M.

The slag detection device 100 includes an image capturing unit 1 which approximately horizontally captures an image of the molten steel flow F approximately vertically directed from the tapping hole 31 of the converter 3 toward the ladle 4 and an image processor 2 which is connected to the image capturing unit 1.

For example, a thermal imaging camera (thermography) having a main sensitivity in an infrared light region, a CCD camera having a main sensitivity in a visible light region, or the like can be used as the image capturing unit 1. As these thermal imaging camera (thermography) and the CCD camera, for example, commercially available cameras can be used.

In the present embodiment, a thermal imaging camera having a main sensitivity in an infrared light region is used as the image capturing unit 1. In addition, in a case where the thermal imaging camera (thermography) is used like the present embodiment, a value of a temperature or density (density before converting into a temperature) of a pixel region in a captured image can be calculated. Meanwhile, in a case where the CCD camera is used, a value of a density of the pixel region can be calculated.

For example, the image processor 2 is constituted by a general-purpose personal computer in which a predetermined program is executed for a histogram creation step ST2 or the like described later is installed. In addition, the image processor 2 has a monitor for displaying the captured image obtained by the image capturing unit 1.

The slag detection method according to the present embodiment is performed using the slag detection device 100. Hereinafter, the slag detection method according to the present embodiment will be described.

<Slag Detection Method of Present Embodiment>

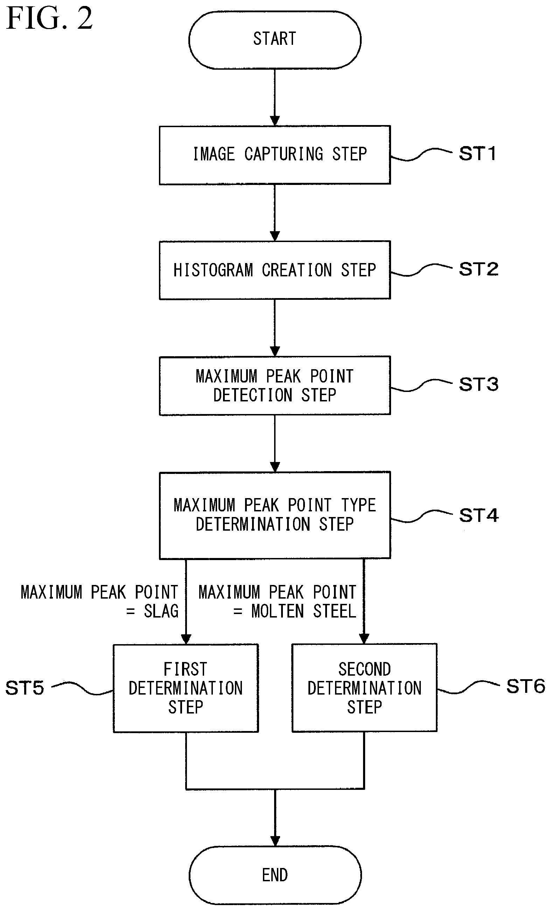

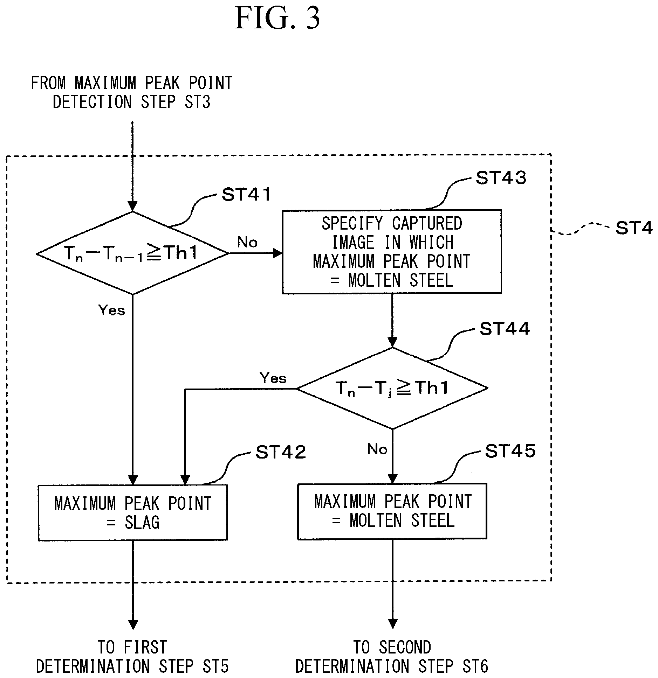

FIG. 2 is a flowchart showing a schematic procedure of the slag detection method according to the present embodiment. In addition, FIG. 3 is a flowchart showing a schematic procedure of a maximum peak point type determination step ST4 shown in FIG. 2.

The slag detection method according to the present embodiment is a method of detecting the slag S in the molten steel flow F based on a plurality of captured images obtained by sequentially capturing an image of the molten steel flow F, which is directed from the converter 3 toward the ladle 4 and includes the molten steel M and the slag S, by the image capturing unit 1. As shown in FIG. 2, the slag detection method has an image capturing step ST1, the histogram creation step ST2, a maximum peak point detection step ST3, the maximum peak point type determination step ST4, a first determination step ST5, and a second determination step ST6.

Hereinafter, a content of each step will be sequentially described.

(Image Capturing Step ST1)

In the image capturing step ST1, the image of the molten steel flow F directed from the converter 3 toward the ladle 4 is sequentially captured by the image capturing unit 1 so as to acquire the plurality of captured images (refer to FIG. 1).

In the present embodiment, the thermal imaging camera is used as the image capturing unit 1, and thus, a captured image acquired in the image capturing step ST1 is obtained by converting a density of each pixel constituting the captured image into a temperature by a predetermined conversion formula. That is, the captured image acquired in the image capturing step ST1 has a value of a temperature detected for each pixel.

A visual field of the image capturing unit 1 is set to a wide visual field including not only the molten steel flow F but also a background so as not to be affected by fluctuations of an outflow position and spread of the molten steel flow F. Even when the visual field of the image capturing unit 1 is set to include the background, a temperature of the background is lower than a temperature of the molten steel flow F, and thus, in the maximum peak point detection step ST3 described later, the pixel region corresponding to the molten steel flow F and the pixel region corresponding to the background can be identified. In addition, the visual field of the image capturing unit 1 may be adjusted narrowly in advance such that only the image of the molten steel flow F is captured. However, in general, the outflow position and the spread of the molten steel flow F fluctuate to some extent depending on a tilt angle of the converter 3 or the like (depending on a position of the tapping hole 31 or the like). For this reason, adjusting the visual field of the image capturing unit 1 such that only the image of the molten steel flow F is captured in any of the early stage of the tapping, the middle stage of the tapping, and the last stage of the tapping is a laborious task. Accordingly, it is preferable that the visual field of the image capturing unit 1 is set to a wide visual field including the background.

For an image capturing timing of the image capturing unit 1, at least one of the plurality of acquired captured images needs to be a captured image obtained by capturing the image of the molten steel flow F mainly including the molten steel M. That is, at least one captured image needs to be obtained in the early stage of the tapping. The reason will be described later. An image capturing timing other than that is not particularly limited. However, in order to increase a time resolution for detecting the slag S, it is preferable to continuously capture an image for each scanning period (a reciprocal number of a frame rate) set in the image capturing unit 1.

In addition, regarding the above-described image capturing timing, if an amount of the slag and an amount of the molten steel in the converter 3 can be estimated and the converter 3 is tilted to some extent, whether or not the outflow of the molten steel flow F mainly including the molten steel M is performed can be geometrically estimated. In addition, whether or not the molten steel flow F mainly includes the molten steel M can be confirmed visually. Based on these, it is possible to capture the image of the molten steel flow F which is discharged from the tapping hole 31 and mainly includes the molten steel M.

The plurality of captured images obtained by the image capturing unit 1 are stored in the image processor 2.

(Histogram Creation Step ST2)

In the histogram creation step ST2, the image processor 2 performs image processing on each of the plurality of captured images acquired in the image capturing step ST1, and a histogram is created, in which a density parameter corresponding to a density of each pixel constituting each captured image is shown on a horizontal axis and the number of pixels which is a total number of pixels each having this density parameter is shown on a vertical axis. The histogram may be created for each captured image or may be created for an average image obtained by averaging a plurality of continuous captured images. In a case where the average image is used, it is preferable to average the plurality of captured images which are continuous within a time (= L/V) obtained by dividing a length L of a pixel region corresponding to the molten steel flow F in the visual field of the image capturing unit 1 by a velocity V of the molten steel flow F.

As the density parameter, a temperature can be exemplified in addition to the density. In a case where the image capturing unit 1 is a thermal imaging camera as in the present embodiment, it is possible to create a histogram whose horizontal axis is a temperature or a density (density before converting to a temperature). Meanwhile, in a case where the image capturing unit 1 is a CCD camera, it is possible to create a histogram whose horizontal axis is a density.

Here, for example, the "density" in the present specification refers to brightness and darkness (that is, the brightness on the image) of an image of 256 gradations. In addition, a relationship between this density and thermal radiation brightness in the molten steel flow is in a linear relationship.

As described above, since the thermal imaging camera is used as the image capturing unit 1 in the present embodiment, a temperature is used as the density parameter (that is, in the present embodiment, the horizontal axis of the histogram is a temperature).

In the present embodiment, as described above, the visual field of the image capturing unit 1 is set to include not only the molten steel flow F but also the background. Therefore, when the histogram is created, the image processor 2 determines that a pixel region having a temperature equal to or higher than a predetermined threshold value (for example, 1000°C) in the captured image is a pixel region corresponding to the molten steel flow F and create a histogram for this pixel region (that is, the histogram creation is not applied to a pixel region whose temperature, which is the horizontal axis, is less than the predetermined threshold value). Accordingly, it is possible to avoid an influence of the background on the histogram (the number of pixels corresponding to the background does not become an absolute maximum value).

In addition, the image processor 2 may create a histogram for all the captured images including the pixel regions corresponding to the background and may avoid the influence of the background by excluding a temperature less than the predetermined threshold value (for example, 1000°C) from a detection range of a maximum peak point in the maximum peak point detection step ST3 described later.

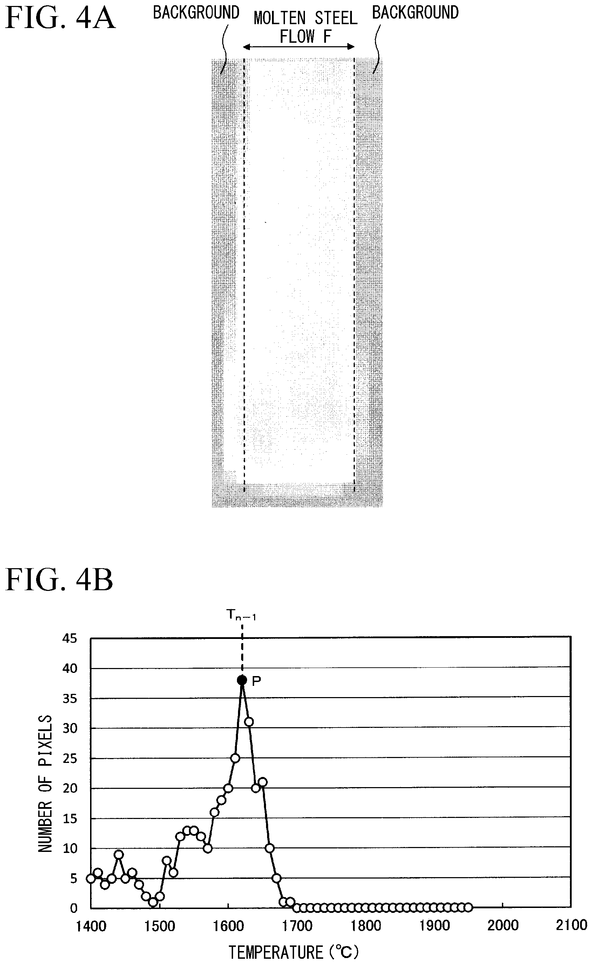

FIGS. 4A to 5B are views and graphs showing an example of the captured image acquired in the image capturing step ST1 and an example of the histogram created in the histogram creation step ST2.

Specifically, for example, FIG. 4A is a view showing an example of the captured image of the molten steel flow F mainly including the molten steel M acquired in the image capturing step ST1 in the early stage of the tapping. Moreover, FIG. 4A is an average image obtained by averaging five captured images continuously acquired for each scanning period of the image capturing unit 1 (the resolution of the captured image is about 3 cm/pixel).

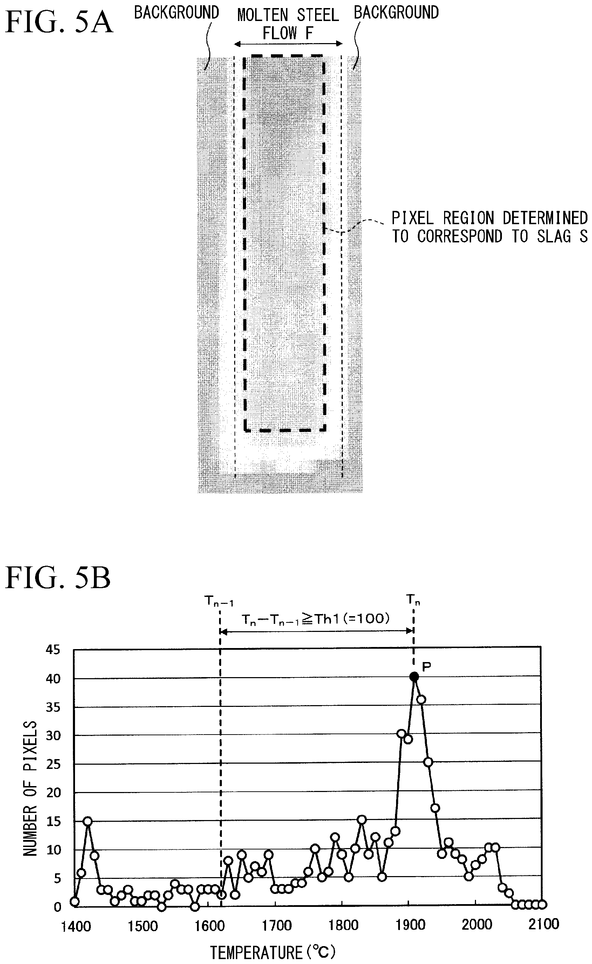

In addition, for example, FIG. 5A is a view showing an example of the captured image of the molten steel flow F mainly including the slag S acquired in the image capturing step ST1 in the middle stage of the tapping. Moreover, FIG. 5A is an average image obtained by averaging five captured images (five captured images continuously acquired after the five captured images which are the origin of FIG. 4A) continuously acquired for each scanning period of the image capturing unit 1 (the resolution of the captured image is about 3 cm/pixel). In addition, in FIG. 5A, a pixel region surrounded by thick dotted lines is a region of a pixel determined to correspond to the slag S in the first determination step ST5 described later, and is a pixel region in which the slag S is considered to exist.

In FIGS. 4A and 5A, only the pixel region corresponding to the molten steel flow F in the captured image (average image) and the pixel region corresponding to the background positioned in the vicinity thereof are partially cut out and displayed. That is, in the captured image to be actually acquired, the pixel region in a right-left direction on the paper surface is wider than in the captured image shown in FIGS. 4A and 5A.

In addition, although the captured image shown in FIGS. 4A and 5A is displayed in monochrome for the convenience of illustration, in actual, different colors depending on a temperature of each pixel are added and displayed on the monitor included in the image processor 2. That is, since the temperature of the pixel region corresponding to the molten steel flow F is higher than the temperature of the pixel region corresponding to the background, in an actual captured image obtained in the image capturing step ST1, the color corresponding to the high temperature is colored.

Moreover, among pixel regions corresponding to the molten steel flow F, a temperature (apparent temperature) of a pixel region (the region surrounded by the thick dotted lines in FIG. 5A) in which the slag S shown in FIG. 5A exists is higher than a temperature (apparent temperature) of a pixel region in which substantially only the molten steel M shown in FIG. 4A exists, and a color corresponding to the high temperature is colored the pixel region in which the slag S exists. In addition, in the molten steel flow F discharged from the converter 3, it is considered that an actual temperature of the region corresponding to the pixel region in which the slag S exists and an actual temperature of the region corresponding to the pixel region in which only the molten steel M substantially exists are equivalent to each other. The reason for this is that, as described above, a temperature dispersion is suppressed by stirring due to upper blowing or upper-bottom blowing during refining, and the molten steel M and slag S in the converter 3 are discharged out of the converter 3 as the molten steel flow F.

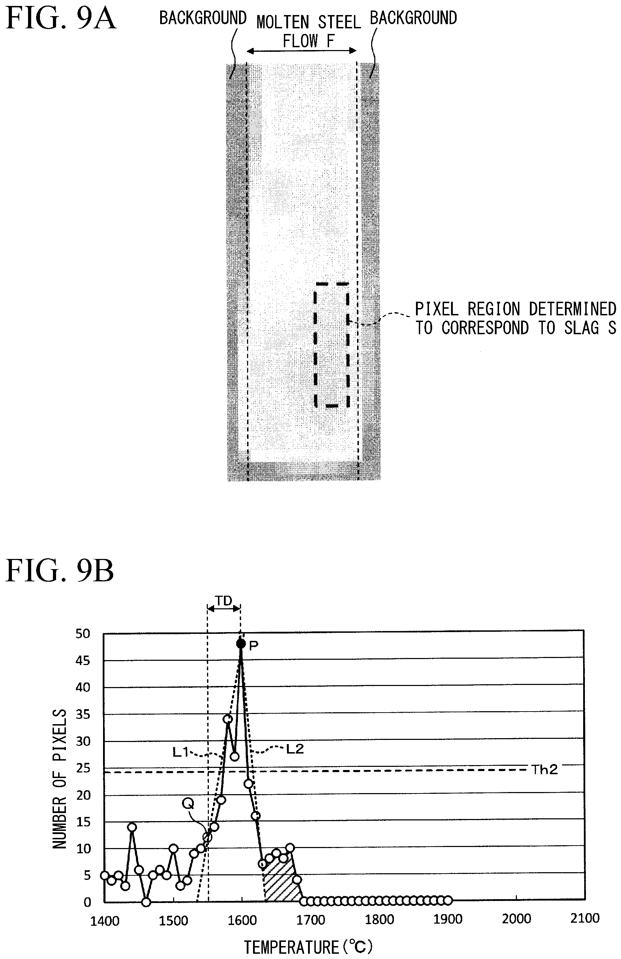

Meanwhile, as described above, since the emissivity of the slag S is higher than the emissivity of the molten steel M, in a case where the emissivity in the image capturing unit 1 is set to the same value for any pixel, in the acquired captured image, the temperature of the pixel region in which the slag S exists is measured higher than the temperature of the pixel region in which substantially only the molten steel M exists. The same is applied to FIG. 9A described later.

FIG. 4B is a graph showing a histogram created for the captured image (average image) shown in FIG. 4A. When the histogram of FIG. 4B is created, in order to avoid the influence of the background, a temperature range of the horizontal axis is set to the predetermined threshold value (1000°C) or more (However, illustration is omitted for temperatures below 1400°C, where no feature is found in a distribution of the number of pixels), the horizontal axis is divided by a 10°C pitch, and the vertical axis is the number of pixels having the temperature of each division.

FIG. 5B is a graph showing a histogram created in the same manner as FIG. 4B for the captured image (average image) shown in FIG. 5A.

(Maximum Peak Point Detection Step ST3)

In the maximum peak point detection step ST3, the image processor 2 detects a maximum peak point, which is an absolute maximum value of the number of pixels, for each histogram created in the histogram creation step ST2. In the histogram shown in FIG. 4B and FIG. 5B, a point indicated by a reference symbol P is the maximum peak point.

Hereinafter, the captured image shown in FIG.

5A is defined as an nth ($n \geq 2$: n is a natural number of 2 or more) captured image among the plurality of captured images acquired by sequentially capturing the image of the molten steel flow F in the image capturing step ST1, the maximum peak point in the histogram shown in FIG. 5B created for the captured image shown in FIG. 5A is defined as $P_n$, and a temperature of $P_n$ is defined as $T_n$. In addition, the captured image (average image) shown in FIG. 4A is defined as an n-1 captured image (that is, a captured image immediately before (a previous image of) the captured image of FIG. 5A), the maximum peak point in the histogram shown in FIG. 4B created for the captured image shown in FIG. 4A is defined as $P_{n-1}$, and a temperature of $P_{n-1}$ is defined as $T_{n-1}$.

(Maximum Peak Point Type Determination Step ST4)

In the maximum peak point type determination step ST4, the image processor 2 sequentially determines to which of the slag S and the molten steel M discharged as the molten steel flow F the maximum peak point P in each histogram detected in the maximum peak point detection step ST3 corresponds.

For example, a method of determining to which of the slag S and the molten steel M the maximum peak point P ($P_n$) in the histogram shown in FIG. 5B corresponds will be described with reference to FIG. 3 (that is, a case of determining the captured image of FIG. 5A will be described). As shown in FIG. 3, it is determined whether or not a variation $\Delta T$ (a variation $T_n - T_{n-1}$ of the temperature $T_n$ in the histogram of FIG. 5B with respect to the temperature $T_{n-1}$ in the histogram of FIG. 4A) obtained by subtracting the temperature $T_{n-1}$ at the maximum peak point P ($P_{n-1}$) in the histogram of FIG. 4B from the temperature $T_n$ at the maximum peak point P ($P_n$) of the histogram of FIG. 5B is equal or more than a predetermined value Th1 (for example, 100°C) (Step ST41 of FIG. 3). In addition, in a case where this variation $\Delta T$ is equal to or more than the predetermined value Th1, it is determined that the maximum peak point P in the histogram of FIG. 5B corresponds to the slag S existing in the molten steel flow F (Step ST42 of FIG. 3).

In addition, as shown in FIG. 5B, since the variation $\Delta T$ ($T_n - T_{n-1}$) of the temperature $T_n$ in the histogram of FIG. 4B with respect to the temperature $T_{n-1}$ in the histogram of FIG. 4A is equal to or more than the predetermined value Th1, it is determined that the maximum peak point P in the histogram of the captured image shown in FIG. 5A (the histogram of FIG. 5B) corresponds to the slag S existing in the molten steel flow F.

Meanwhile, in a case where the variation $\Delta T$ is less than the predetermined value Th1 (a case where, for example, the captured image of the molten steel flow F mainly including the molten steel M is set to a determination target as the captured image of FIG. 5A and the variation $\Delta T$ is less than the predetermined value Th1 is assumed), whether or not the maximum peak point P corresponds to the slag S existing in the molten steel flow F is not immediately determined, and among the captured images acquired before the captured image of FIG. 5A, a captured image is specified in which the maximum peak point P is determined to correspond to the molten steel M existing in the molten steel flow F (Step ST43 in FIG. 3). That is, in this case, a captured image (a jth ($j \leq$ n-1: j is a natural number equal to or less than n-1) captured image), which is acquired before the nth captured image and is one captured image of the captured images in which the maximum peak point P is determined to correspond to the molten steel M, is specified as a reference image (that is, the jth ($j \leq$ n-1: j is a natural number equal to or less than n-1) captured image which is acquired before the nth captured image and in which the maximum peak point $P_j$ is determined to correspond to the molten steel M is specified as the reference image).

Subsequently, it is determined whether or not a variation $\Delta T'$ ($T_n - T_j$) of the temperature $T_n$ at the maximum peak point P ($P_n$) with respect to the temperature $T_j$ at the maximum peak point P ($P_j$) in the histogram of the reference image is equal to or more than the predetermined value Th1 (Step S44 in FIG. 3). In addition, if the variation $\Delta T'$ ($T_n - T_j$) is equal to or more than the predetermined value Th1, it is determined that the maximum peak point P ($P_n$) corresponds to the slag S existing in the molten steel flow F (Step ST42 in FIG. 3). Meanwhile, if the variation $\Delta T'$ ($T_n - T_j$) is less than the predetermined value Th1, it is determined that the maximum peak point P ($P_n$) corresponds to the molten steel M existing in the molten steel flow F (Step ST45 in FIG. 3).

If Step ST43 in FIG. 3 is described in more detail, in the case of the captured image of the molten steel flow F mainly including the molten steel M as in FIG. 4A, it is determined that the maximum peak point P in the histogram corresponds to the molten steel M existing in the molten steel flow F. Accordingly, in Step ST43, for example, the captured image of FIG. 4A is specified as the reference image, and the subsequent Step ST44 is performed. In addition, it is not necessary to specify the captured image of FIG. 4A as the reference image, and in a case where there is a captured image which is acquired before FIG. 4A and is determined as the maximum peak point P corresponding to the molten steel M existing in the molten steel flow F, the captured image may be specified as the reference image, and the subsequent Step ST44 may be performed.

However, it is preferable that the reference image used to calculate the variation $\Delta T$ is closer in imaging capturing order to the captured image which is the determination target. As the reference image is closer in imaging capturing order, a temperature dispersion of the molten steel flow F decreases, and it is possible to more accurately detect the slag. Accordingly, in Step ST43, in a case where the captured image of FIG. 5A is determined, it is preferable that the captured image of FIG. 4A, which is the captured image acquired immediately before FIG. 5A and is determined as the maximum peak point corresponding to the molten steel M, is specified as the reference image.

Here, as described above, the maximum peak point type determination step ST4 uses a difference between the temperature $T_n$ at the maximum peak point $P_n$ in the histogram of the captured image which is the determination target and the temperature $T_{n-1}$ at the maximum peak point $P_{n-1}$ in the histogram of the captured image immediately before the captured image which is the determination target, in Step ST41. Accordingly, in the first captured image (the captured image which is initially acquired), it is not possible to determine the maximum peak point using the above-described method.

As described above, in the early stage of the tapping, whether or not the molten steel flow F mainly includes the molten steel M can be confirmed by the amount of the slag and the amount of the molten steel in the converter 3, the tilt angle of the converter 3 at the time of the tapping, a visual confirmation, or the like. Accordingly, based on these, at least one image of the molten steel flow F when only the molten steel M is mainly discharged from the tapping hole 31 is captured, and it is determined that a maximum peak point of this captured image corresponds to the molten steel M. In addition, if it can be confirmed that the molten steel flow F mainly includes the molten steel M, in the early stage of the tapping, a plurality of captured images may be acquired, and it may be determined that the maximum peak points of the captured images correspond to the molten steel M.

As described above, in the maximum peak point type determination step ST4, the determination is not performed using only the temperature at the maximum peak point of the captured image which is the determination target, and the determination is performed using the temperature at the maximum peak point of the captured image which is the determination target and the variation with respect to the temperature at the maximum peak point of the captured image acquired before the captured image which is the determination target. Accordingly, even in a case where a condition of a tapping operation or the like is changed, it is possible to accurately detect the slag.

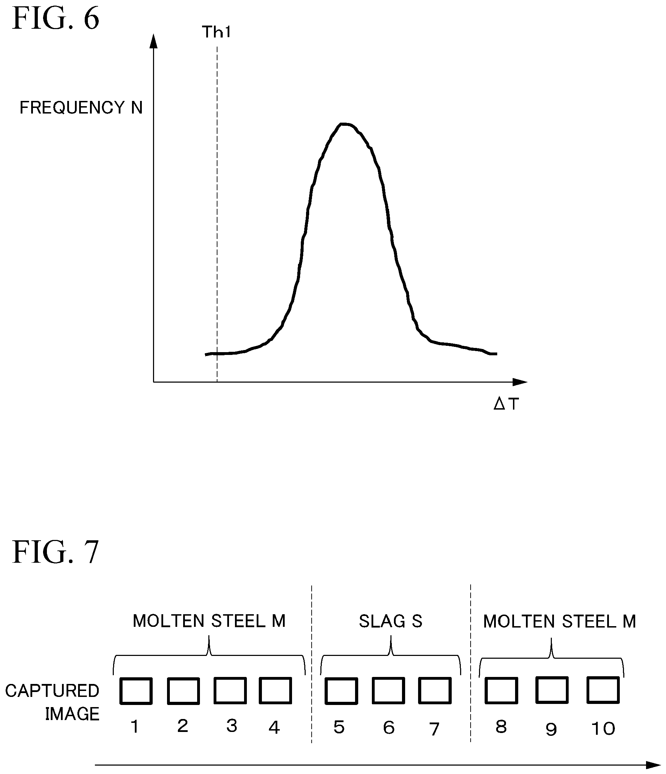

Here, an example of a determination method of the predetermined value Th1 will be described. The emissivity of the molten steel M and the slag S varies somewhat according to components thereof. Accordingly, it is preferable to more accurately detect the slag even in a case where components of the molten steel and the slag in the converter are changed. From the viewpoint of this, for example, a graph shown in FIG. 6 is created, in which image of various molten steel flows including the molten steel and the slag having different components thereof are captured, a histogram for each molten steel flow is created, the variation $\Delta T$ between the molten steel peak and the slag peak in each histogram is calculated, and thereafter, $\Delta T$ is shown in a horizontal axis and a frequency N is shown on a vertical axis. In addition, in the graph shown in FIG. 6, for example, a value near a minimum value of the variation $\Delta T$ is determined as the predetermined value Th1.

Subsequently, the maximum peak point type determination step ST4 will be described in more detail with reference to FIG. 7. FIG. 7 is a view for explaining the maximum peak point type determination step ST4, and is a schematic view showing an example of the plurality of captured images obtained in the image capturing step ST1.

As shown in FIG. 7, ten captured images of the molten steel flow F in the image capturing step ST1 are acquired. Among these captured images, first to fourth captured images are obtained by capturing the image of the molten steel flow F which mainly includes the molten steel M, fifth to seventh captured images are obtained by capturing the image of the molten steel flow F which mainly includes the slag S, and eighth to tenth captured images are obtained by capturing the image of the molten steel flow which mainly includes the molten steel M. In addition, a determination method of the maximum peak points of the captured images will be described below.

As described above, in the first captured image, it is determined that the maximum peak point corresponds to the molten steel M.

Subsequently, in the second captured image, according to Step ST41 (refer to FIG. 3) of the maximum peak point type determination step ST4, the variation $\Delta T$ is calculated by subtracting the temperature $T_1$ at the maximum peak point $P_1$ of the first captured image acquired immediately before from the temperature $T_2$ at the maximum peak point $P_2$ of the second captured image which is the present determination target (Step ST41 in FIG. 3). The second captured image is obtained by capturing the image of the molten steel flow F mainly including the molten steel M, the variation $\Delta T$ is less than the predetermined value Th1, and the first captured image in which the maximum peak point is determined as the molten steel M is specified as the reference image (Step ST43 in FIG. 3). In addition, in Step ST44 of FIG. 3, the variation $\Delta T'$ ($T_n - T_j$) is less than the predetermined value Th1, and thus, it is determined that the maximum peak point of the second captured image corresponds to the molten steel M.

In the third captured image, similarly to the case of the second captured image, the variation $\Delta T$ is calculated by subtracting the temperature $T_2$ at the maximum peak point $P_2$ of the second captured image acquired immediately before from the temperature $T_3$ at the maximum peak point $P_3$ of the third captured image which is the present determination target. The third captured image is obtained by capturing the image of the molten steel flow F mainly including the molten steel M, the variation $\Delta T$ is less than the predetermined value Th1, and the first captured image or the second captured image in which the maximum peak point is determined to correspond to the molten steel M is specified as the reference image. In addition, in Step ST44 of FIG. 3, the variation $\Delta T'$ ($T_n - T_j$) is less than the predetermined value Th1, and thus, it is determined that the maximum peak point of the third captured image corresponds to the molten steel M. In addition, the first captured image may be used as the reference image. However, it is preferable that the second captured image closest in image capturing order (acquisition order) to the third captured image is used as the reference image. As the reference image is closer in imaging capturing order, the temperature dispersion decreases, and it is possible to more accurately detect the slag.

In the fourth captured image, the determination is performed similarly to the case of the third capture image.

In the fifth captured image, the variation $\Delta T$ is calculated by subtracting the temperature $T_4$ at the maximum peak point $P_4$ of the fourth captured image acquired immediately before from the temperature $T_5$ at the maximum peak point $P_5$ of the fifth captured image which is the present determination target. The fifth captured image is obtained by capturing the image of the molten steel flow F mainly including the slag S and the fourth captured image is obtained by capturing the image of the molten steel flow F mainly including the molten steel M, and thus, the variation $\Delta T$ is equal to or more than the predetermined value Th1, and it is determined that the maximum peak point of the fifth captured image corresponds to the slag S.

In the sixth captured image, the variation $\Delta T$ is calculated by subtracting the temperature $T_5$ at the maximum peak point $P_5$ of the fifth captured image acquired immediately before from the temperature $T_6$ at the maximum peak point $P_6$ of the sixth captured image which is the present determination target. The sixth captured image is obtained by capturing the image of the molten steel flow F mainly including the slag S and the fifth captured image is obtained by capturing the image of the molten steel flow F mainly including the slag S, and thus, the variation $\Delta T$ is less than the predetermined value Th1. Accordingly, according to the subsequent Step ST43 (refer to FIG. 3), any one of the first to fourth captured images in which the maximum peak point is determined to correspond to the molten steel M is specified as the reference image. In addition, in Step ST44 of FIG. 3, the variation $\Delta T'$ ($T_n - T_j$) is equal to or more than the predetermined value Th1, and thus, it is determined that the maximum peak point of the sixth captured image corresponds to slag S.

In the seventh captured image, the determination is performed similarly to the case of the sixth captured image.

In the eighth captured image, the variation $\Delta T$ is calculated by subtracting the temperature $T_7$ at the maximum peak point $P_7$ of the seventh captured image acquired immediately before from the temperature $T_8$ at the maximum peak point $P_8$ of the eighth captured image which is the present determination target. The eighth captured image is obtained by capturing the image of the molten steel flow F mainly including the molten steel M and the seventh captured image is obtained by capturing the image of the molten steel flow F mainly including the slag S, and thus, the variation $\Delta T$ is less than the predetermined value Th1. Accordingly, according to the subsequent Step ST43 (refer to FIG. 3), any one of the first to fourth captured images in which the maximum peak point is determined to correspond to the molten steel M is specified as the reference image. In addition, in Step ST44 of FIG. 3, the variation $\Delta T'$ ($T_n - T_j$) is less than the predetermined value Th1, and thus, it is determined that the maximum peak point of the eighth captured image corresponds to the molten steel M.

In the ninth captured image, the variation $\Delta T$ is calculated by subtracting the temperature $T_8$ at the maximum peak point $P_8$ of the eighth captured image acquired immediately before from the temperature $T_9$ at the maximum peak point $P_9$ of the ninth captured image which is the present determination target. The ninth captured image is obtained by capturing the image of the molten steel flow F of the molten steel M and the eighth captured image is obtained by capturing the image of the molten steel flow F of the molten steel M, and thus, the variation $\Delta T$ is less than the predetermined value Th1. Accordingly, according to the subsequent Step ST43 (refer to FIG. 3), any one of the first to fourth captured images and the eighth captured image in which the maximum peak point is determined to correspond to the molten steel is specified as the reference image. In addition, in Step ST44 of FIG. 3, the variation $\Delta T'$ ($T_n - T_j$) is less than the predetermined value Th1, and thus, it is determined that the maximum peak point of the ninth captured image corresponds to the molten steel M.

In the tenth captured image, the determination is performed similarly to the case of the ninth captured image.

As described above, in the maximum peak point type determination step ST4, the above-described determinations are sequentially performed on the plurality of captured images acquired in the image capturing step ST1.

In addition, in the example shown in FIG. 7, Step ST41 of FIG. 3 is performed from the second captured image. However, as described above, for example, if it is possible to visually confirm that the molten steel flow F corresponds to the molten steel M in the early stage of the tapping, similarly to the first captured image, Step ST41 of FIG. 3 is not performed on the second captured image, and the maximum peak point P may be determined to correspond to the molten steel M.

(First Determination Step ST5)

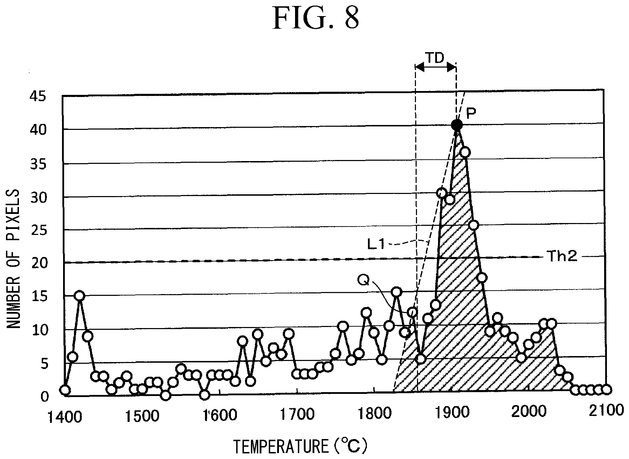

The first determination step ST5 is performed based on a determination result of the maximum peak point type determination step ST4. Specifically, in a case where it is determined that the maximum peak point P of the captured image which is the determination target corresponds to the slag S existing in the molten steel flow F, the first determination step ST5 is performed by the image processor 2. If it is described with FIG. 5B (the captured image of the molten steel flow F mainly including the slag S) as an example, in the first determination step ST5, the image processor 2 determines that, among images constituting the captured image, a pixel having a temperature less than a first threshold value determined based on the maximum peak point P corresponds to the molten steel M existing in the molten steel flow F and a pixel having a temperature equal to or more than the first threshold value corresponds the slag S existing in the molten steel flow F. Hereafter, it is described in more detail with reference to FIG. 8 appropriately.

FIG. 8 is a graph for explaining the first threshold value determined in the first determination step ST5. In addition, a histogram shown in FIG. 8 is the same as the histogram shown in FIG. 5B.

As shown in FIG. 8, the first threshold value is represented by a first straight line L1 which passes through the maximum peak point P and has a positive inclination in the histogram created in the histogram creation step ST2. Specifically, the first straight line L1 is a straight line which passes through a point Q and the maximum peak point P shown in FIG. 8. The point Q is a peak point having a highest temperature among from points having the number of pixels less than a predetermined threshold value Th2 of the number of pixels (for example, 50% of the number of pixels of the maximum peak point P) and a temperature less than the temperature at the maximum peak point P by a predetermined value TD (for example, 50°C) or more (that is, the point Q is a point having the highest temperature among points which have the number of pixels less than a predetermined threshold value Th2 of the number of pixels and the temperature less than the temperature at the maximum peak point P by the predetermined value TD or more and are local maximum values).

Here, in a case where it is determined whether or not the point having the highest temperature from among points having the lower temperature by the predetermined value TD or more is a peak point which is a determination target, focusing on a gradient of a line connecting the point and a point adjacent to a low temperature side of the point to each other, if the line has a positive inclination (the line is a line rising to a right), the point of the determination target is regarded as the point Q.

In addition, the slag detection method according to the present embodiment is particularly preferably applied to a molten steel flow in which the number of peaks exceeding the predetermined threshold value Th2 of the number of pixels in the histogram is small. In addition, for example, the slag detection method according to the present embodiment is particularly preferably applied to a molten steel flow in which a peak having 50% or more of the number of pixels of the maximum peak point P has two points or less, regardless of a presence or absence of setting of the predetermined threshold value Th2 of the number of pixels. Characteristics of the peak are determined by a mixing condition between the molten steel and the slag in scouring.

When the temperature of the horizontal axis is defined as X and the number of pixels of the vertical axis is defined as Y, the first threshold value (first straight line L1) is expressed by the following Expression (1).

$$Y = aX + b \ldots(1)$$

Here, a is a positive constant and b is a constant.

The constants are determined from the first straight line L1 passing through the point Q and the maximum peak point P.

The predetermined value TD is not particularly limited. However, for example, the predetermined value TD is 50°C. In experience, in most cases, a range within ±50°C of the maximum peak temperature does not fall on a base. Accordingly, for example, by setting the predetermined value TD to 50°C, preferably, the first threshold value can be determined by the peak excluding the base.

In addition, the predetermined threshold value Th2 of the number of pixels is not particularly limited. However, for example, preferably, 50% of the number of pixels of the maximum peak point P are set to Th2 such that a peak of a temperature range considered to be the background such as 1200°C to 1300°C is not ascertained.

As described above, the image processor 2 determines that the pixel having the temperature less than the first threshold value corresponds to the molten steel M existing in the molten steel flow F. That is, it is determined that the pixel satisfying Y > aX + b corresponds to the molten steel M existing in the molten steel flow F.

Meanwhile, the image processor 2 determines that the pixel having the temperature equal to or more than the first threshold value corresponds to the slag S existing in the molten steel flow F. That is, in the present embodiment, it is determined that the pixel (pixel corresponding to a hatched region in FIG. 8) satisfying Y ≤ aX + b corresponds to the slag S existing in the molten steel flow F.

(Second Determination Step ST6)

The second determination step ST6 is performed based on the determination result of the maximum peak point type determination step ST4. Specifically, in the maximum peak point type determination step ST4, in a case where it is determined that the maximum peak point P of the captured image which is the determination target corresponds to the molten steel M existing in the molten steel flow F, the second determination step ST6 is performed by the image processor 2. In the second determination step ST6, the image processor 2 determines that, among the pixels constituting the captured image, a pixel having a temperature equal to or less than a second threshold value determined based on the maximum peak point P corresponds to the molten steel M existing in the molten steel flow F and a pixel having a temperature higher than the second threshold value corresponds the slag S existing in the molten steel flow F. Hereafter, it is described in more detail with reference to FIGS. 9A and 9B appropriately.

FIG. 9A is a captured image acquired in the image capturing step ST1 and is a view showing an example different from those of FIGS. 4A and 5A. In addition, FIG. 9A shows an average image obtained by averaging five captured images continuously acquired for each scanning period of the image capturing unit 1. An image capturing condition or the like is similar to those of FIGS. 4A and 5A.

Moreover, FIG. 9B is a graph showing a histogram created based on the captured image of FIG. 9A and is a graph for explaining the second threshold value determined in the second determination step ST6.

In the example shown in FIG. 9B, according to the above-described maximum peak point type determination step ST4, it is determined that the maximum peak point P in the histogram corresponds to the molten steel M existing in the molten steel flow F. Accordingly, the image processor 2 performs the second determination step ST6.

As shown in FIG. 9B, the second threshold value is represented by a second straight line L2 which passes through the maximum peak point P and has a negative inclination. An absolute value of the inclination of the second straight line L2 is larger than an absolute value of the inclination of the first straight line L1 (preferably, the absolute value of the inclination of the second straight line L2 is 1.5 to 2.5 times the absolute value of the inclination of the first straight line L1). The first straight line L1 is the straight line which passes through the point Q and the maximum peak point P shown in FIG. 9B. In addition, a gradient of a line between the point Q and a point adjacent to a low temperature side is positive, and thus, similarly to FIG. 8, the point Q is the peak point having the highest temperature among from the points having the number of pixels less than the threshold value Th2 of the number of pixels and a temperature less than the temperature at the maximum peak point P by the predetermined value TD (for example, 50°C) or more.

As described above, when the temperature of the horizontal axis is defined as X and the number of pixels of the vertical axis is defined as Y, the first straight line L1 is expressed by the following Expression (1).

$$Y = aX + b \ \dots(1)$$

Here, a is a positive constant and b is a constant. The constants are determined from the first straight line L1 passing through the point Q and the maximum peak point P.

Meanwhile, for example, if the absolute value of the inclination of the second straight line L2 is set to two times the absolute value of the inclination a of the first straight line L1, the second straight line L2 is expressed by the following Expression (2).

$$Y = -2aX + c \ \dots(2)$$

Here, a is a positive constant and c is a constant. In addition, a is determined from the first straight line L1 and c is determined from the second straight line passing through the maximum peak point P.

As described above, the image processor 2 determines that the pixel having the temperature equal to or less than the second threshold value corresponds to the molten steel M existing in the molten steel flow F. That is, in the histogram shown in FIG. 9B, it is determined that the pixel satisfying $Y \leq -2aX + c$ corresponds to the molten steel M existing in the molten steel flow F.

Meanwhile, the image processor 2 determines that the pixel having the temperature higher than the second threshold value corresponds to the slag S existing in the molten steel flow F. That is, in the histogram shown in FIG. 9B, it is determined that the pixel (pixel corresponding to a hatched region in FIG. 9B) satisfying $Y > -2aX + c$ corresponds to the slag S existing in the molten steel flow F.

As described above, according to the slag detection method of the present embodiment, based on the difference between the temperature in the maximum peak point of the captured image which is the determination target and the temperature in the maximum peak point of the captured image obtained before the captured image which is the determination target, whether the maximum peak point of the captured image which is the determination target corresponds to the molten steel M or the slag S is determined. Accordingly, even in the case where the condition of the tapping operation or the like is changed and thus, the temperature of the molten steel flow F is changed, it is possible to accurately perform the detection. Therefore, it is possible to accurately detect the slag S in the molten steel flow F.

In addition, according to the slag detection method of the present embodiment, in the first determination step ST5 or the second determination step ST6, it is possible to calculate the number of pixels (area) corresponding to the slag S existing in the molten steel flow F and the number of pixel (area) corresponding to the molten steel M existing in the molten steel flow F. Accordingly, for example, it is possible to an area ratio of the slag S in the molten steel flow F and a volume ratio of the slag S in the molten steel flow F. In addition, if specific gravity of each of the molten steel M and the slag S is used, it is possible to calculate a mass ratio of the slag S in the molten steel flow F, and a flow rate of the molten steel flow F can be estimated from the tilt angle of the converter 3 at the time of tapping. Accordingly, it is possible to estimate an outflow amount (flow rate) of the slag S using the mass ratio of the slag S and the flow rate of the molten steel flow F, and the outflow amount of the slag S can be controlled to a range required in the tapping operation of the converter 3.

Specifically, according to the slag detection method of the present embodiment, the following controls can be performed. That is, the tapping operation ends in a case where the outflow amount or the like (the outflow amount, the number of pixels, the area, the volume, or the like) of the slag S is larger than zero, the tapping operation ends in a case where the outflow amount or the like of the slag S is larger than a predetermined value, the tapping operation ends in a case where a ratio of the outflow amount or the like of the slag S with respect to the outflow amount or the like of the molten steel M is larger than a predetermined value, or the like.

[Example]

Next, Example carried out to confirm operation and effect of the present invention will be described.

The slag detection method according to the present embodiment and the slag detection method described in Patent Document 2 were compared with each other using the captured image shown in FIG. 5A as an evaluation target.

Specifically, in the slag detection method according to the present embodiment, as described above, in the histogram shown in FIG. 5B created for the captured images shown in FIG. 5A, it is determined that the maximum peak point P corresponds to the slag S existing in the molten steel flow F. In addition, as shown in FIG. 8, it is determined that the pixels corresponding to the hatched region correspond to the slag S by the first straight line L1 expressed by Expression (1).

In the example shown in FIG. 8, it was determined that 309 pixels correspond to the slag S.

Meanwhile, if the slag detection method disclosed in Patent Document 2 is used, the maximum peak point P in the histogram shown in FIG. 5B is considered to correspond to the molten steel M existing in the molten steel flow F. As described above, according to the slag detection method disclosed in Patent Document 2, the pixel having the density value of N1 or more considering the standard deviation $\sigma$ in the horizontal axis direction of the maximum peak point P is determined as the molten steel M, and the pixel having the density value of N2 or more obtained by adding the bias value B to the density value N1 is determined as the slag S. If the density value is replaced by a temperature, in the slag detection method described in Patent Document 2, the pixel having a temperature of N1 or more considering the standard deviation $\sigma$ in the horizontal axis direction of the maximum peak point P is determined as the molten steel M, and the pixel having a temperature of N2 or more obtained by adding the bias value B to a temperature N1 is determined as the slag S. Here, in the slag detection method described in Patent Document 2, since it is considered that the maximum peak point P corresponds to the molten steel M, it is appropriate to set the bias value B to $2\sigma$ or more in order to distinguish the pixel corresponding to the molten steel M and the pixel corresponding to the slag S (That is, the temperature N2 is set to the temperature $+ \sigma$ or more of the maximum peak point P). In the present evaluation, the minimum value $2\sigma$ at which a detection error of the slag S becomes the smallest is used as the bias value B. Moreover, in the histogram shown in FIG. 5B, assuming that a pixel number distribution having the temperature or more of the maximum peak point P is a normal distribution, $\sigma$ is set such that a value obtained by dividing a sum of the numbers of pixels from the temperature at the maximum peak point P to the temperature N2 (the temperature at the maximum peak point P $+ \sigma$) by a sum of the number of pixels of the temperature or more of the maximum peak point P is about 68%.

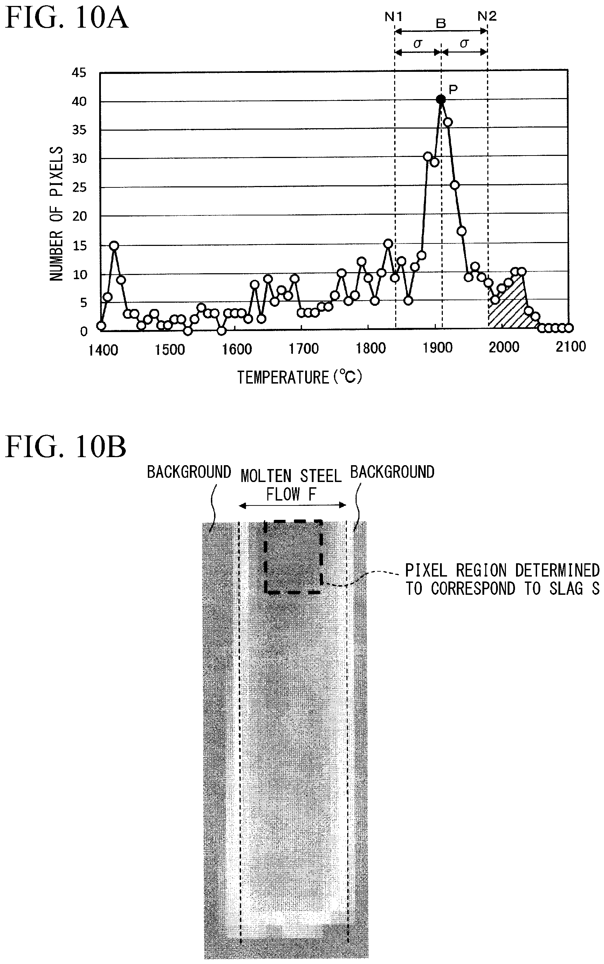

FIGS. 10A and 10B are a graph and a view for explaining the slag detection method described in Patent Document 2. FIG. 10A shows a histogram and FIG. 10B shows a captured image (average image). The histogram shown in FIG. 10A is the same as the histogram shown in FIG. 5B or FIG. 8. The captured image shown in FIG. 10B is the same as the captured image shown in FIG. 5A. According to the slag detection method described in Patent Document 2, it is determined that pixels corresponding to a hatched region in FIG. 10A correspond to the slag S. According to the present evaluation, it was determined that 50 pixels corresponding to a pixel region surrounded by thick dotted lines in FIG. 10B correspond to the slag S.

The number of pixels corresponding to the molten steel flow F in the captured image shown in FIG. 5A was 465. Since the pixel entire region corresponding to the molten steel flow F has a temperature exceeding an operation temperature, if it is assumed that the entire pixel region corresponding to the molten steel flow F corresponds to the slag S, a true value of the number of pixels corresponding to the slag S can be regarded as 465. Accordingly, assuming that 465 is the true value of the number of pixels corresponding to the slag S, there were errors of the true value - 89.2% ((50 - 465)/465 x 100 = -89.2) in the slag detection method described in Patent Document 2 while there were errors of the true value - 33.5% ((309-465)/465 x 100 = - 33.5) in the slag detection method according to the present embodiment. Therefore, compared to the slag detection method described in Patent Document 2, according to the slag detection method of the present embodiment, it is possible to accurately detect the slag S in the molten steel flow F. This is visually clearer in that, compared to the thick dotted lines shown in FIG. 10B, the thick dotted lines shown in FIG. 5A are closer to an outline of the pixel region of the molten steel flow F in which the slag S is considered to exist.

In addition, if the true value of the number of pixels corresponding to the slag S is converted into an area (actual size), an area of one pixel is about $9cm^2$, $9 \times 465 = 4185$ $cm^2$. If this is simply converted into a volume (converted assuming that dimensions of the slag S in a visual axis direction of the image capturing unit 1 are the same as dimensions of the slag S in a visual field plane of the image capturing unit 1), $(4185)^{3/2} = 270734$ $cm^3 = 270734 \times 10^{-6}m^3$. Accordingly, if the specific gravity of the slag S is set to $2 \times 10^{-3}$ $m^3/kg$, the mass of the slag S is $(270734 \times 10^{-6})/(2 \times 10^{-3}) = 135$ kg.

Similarly, if the slag S detected by the slag detection method according to the present embodiment is converted into the mass, the mass is 73 kg (54.1% of the true value), and if the slag S detected by the slag detection method described in Patent Document 2 is converted into the mass, the mass is 5 kg (3.7% of the true value). That is, compared to the method described in Patent Document 2 in which an error of - 96.3% occurs, according to the slag detection method of the present embodiment, an error of -45.9% in the mass occurs, and thus, it is possible to accurately detect the slag S in the molten steel flow F.

Hereinbefore, the embodiment of the present invention is described. However, the embodiment is suggested as an example, and the scope of the present invention is not limited to only the embodiment. The embodiment can be embodiment in other various aspects, and various omissions, replacements, and modifications can be made within the scope which does not depart from the gist of the present invention. The embodiments or the modifications thereof are included in the invention described in the claims and the equivalents thereof as well as included in the scope and the gist of the present invention.

For example, in the above-described embodiment, the case is described, in which the first threshold value is represented by the first straight line L1 which passes through the maximum peak point P and has a positive inclination. With respect to how to determine the threshold value, in order to detect the slag with higher accuracy, it is preferable to perform the determination by performing fitting with a Gaussian distribution or the like on each of the peaks of the molten steel and the slag. However, this method requires a long calculation time, which is not preferable from the viewpoint of industry. Accordingly, it is possible to more easily determine the threshold value by representing the first threshold value as the straight line.

In addition, the present invention is not limited to the case where the first threshold value and the second threshold value are represented by the first straight line L1 and the second straight line L2. For example, each of the first threshold value and the second threshold value may be represented by a straight line (a straight line with an infinite inclination) orthogonal to the horizontal axis in consideration of the dispersion in the horizontal axis direction of the maximum peak point.

[Brief Description of the Reference Symbols]

1: image capturing unit
2: image processor
3: converter
4: ladle
100: slag detection device
ST1: image capturing step
ST2: histogram creation step
ST3: maximum peak point detection step
ST4: maximum peak point type determination step
ST5: first determination step
ST6: second determination step
F: molten steel flow
M: molten steel
S: slag

**Claims**

1. A method of detecting slag in a molten steel flow, comprising:

an image capturing step of sequentially capturing a molten steel flow which is directed from a converter toward a ladle and includes molten steel and slag to acquire a plurality of captured images of the molten steel flow;

a histogram creation step of creating a histogram, in which a density parameter corresponding to a density of each pixel constituting each captured image is shown on a horizontal axis and the number of pixels that is a total number of pixels each having the density parameter is shown on a vertical axis, for each captured image;

a maximum peak point detection step of detecting a maximum peak point, in which the number of pixels is an absolute maximum value, for each histogram; and

a maximum peak point type determination step of determining to which of the slag or the molten steel the maximum peak point of each histogram corresponds,

wherein when a maximum peak point $P_n$ of a histogram of an nth ($n \geq 2$) captured image is determined in the maximum peak point type determination step,

in a case where a variation $\Delta T$ of a density parameter $T_n$ at the maximum peak point $P_n$ with respect to a density parameter $T_{n-1}$ at a maximum peak point $P_{n-1}$ in a histogram of an n-1th captured image is equal to or more than a predetermined value, it is determined that the maximum peak point $P_n$ corresponds to the slag, and in a case where a variation $\Delta T$ is less than the predetermined value, if a variation $\Delta T'$ of the density parameter $T_n$ with respect to a density parameter $T_j$ at a maximum peak point $P_j$ in a histogram of a jth ($j \leq n-1$) captured image, in which a maximum peak point is determined as the molten steel, is equal to or more than the predetermined value, it is determined that the maximum peak point $P_n$ corresponds to the slag, and if a variation $\Delta T'$ is less than the predetermined value, it is determined that the maximum peak point $P_n$ corresponds to the molten steel.

2. The method of detecting slag in a molten steel flow according to claim 1,
wherein the maximum peak point type determination step includes, as the maximum peak point $P_j$, using a maximum peak point in a histogram of a captured image which is acquired before the nth captured image and is closest in acquisition order to the nth captured image, and in which a maximum peak point is determined as the molten steel.

3. The method of detecting slag in a molten steel flow according to claim 1 or 2, further comprising:

a first determination step of, in a case where it is determined that the maximum peak point corresponds to the slag in the maximum peak point type determination step, determining that a pixel having a density parameter less than a first threshold value determined based on the maximum peak point corresponds to the molten steel and a pixel having a density parameter equal to or more than the first threshold value corresponds to the slag; and
a second determination step of, in a case where it is determined that the maximum peak point corresponds to the molten steel in the maximum peak point type determination step, determining that a pixel having a density parameter equal to or less than a second threshold value determined based on the maximum peak point corresponds to the molten steel and a pixel having a density parameter more than the second threshold value corresponds to the slag.

4. The method of detecting slag in a molten steel flow according to claim 3,
wherein the first threshold value is represented by a first straight line, which passes through the maximum peak point and has a positive inclination, in the histogram,
wherein the second threshold value is represented by a second straight line, which passes through the maximum peak point and has a negative inclination, in the histogram, and
wherein an absolute value of an inclination of the second straight line is larger than an absolute value of an inclination of the first straight line.

5. The method of detecting slag in a molten steel flow according to claim 4,
wherein the first straight line is a straight line which passes through a peak point having a maximum density parameter from among points having the number of pixels less than a threshold value of the number of pixels and a density parameter smaller by a predetermined value or more with respect to the density parameter at the maximum peak point, and the maximum peak point, and
wherein the absolute value of the inclination of the second straight line is 1.5 to 2.5 times the absolute value of the inclination of the first straight line.

FIG. 1

FIG. 2

```
                    ┌─────────────┐
                    │    START    │
                    └──────┬──────┘
                           │
                           ▼
         ┌──────────────────────────────┐
         │    IMAGE CAPTURING STEP       │─────── ST1
         └──────────────┬───────────────┘
                        │
                        ▼
         ┌──────────────────────────────┐
         │  HISTOGRAM CREATION STEP      │─────── ST2
         └──────────────┬───────────────┘
                        │
                        ▼
         ┌──────────────────────────────┐
         │    MAXIMUM PEAK POINT         │─────── ST3
         │    DETECTION STEP             │
         └──────────────┬───────────────┘
                        │
                        ▼
         ┌──────────────────────────────┐
         │  MAXIMUM PEAK POINT TYPE      │─────── ST4
         │  DETERMINATION STEP           │
         └──────────────────────────────┘
```

MAXIMUM PEAK POINT = SLAG     MAXIMUM PEAK POINT = MOLTEN STEEL

```
         ┌──────────────┐      ┌──────────────┐
  ST5 ───│    FIRST     │      │    SECOND    │─────── ST6
         │ DETERMINATION│      │ DETERMINATION│
         │    STEP      │      │    STEP      │
         └──────┬───────┘      └──────┬───────┘
                │                     │
                └──────────┬──────────┘
                           │
                           ▼
                    ┌─────────────┐
                    │     END     │
                    └─────────────┘
```

# FIG. 3

FROM MAXIMUM PEAK POINT
DETECTION STEP ST3

ST41

$T_n - T_{n-1} \geqq Th1$

No → ST43

SPECIFY CAPTURED
IMAGE IN WHICH
MAXIMUM PEAK POINT
= MOLTEN STEEL

ST4

Yes

ST44

$T_n - T_i \geqq Th1$

Yes

No

ST42

MAXIMUM PEAK POINT
= SLAG

ST45

MAXIMUM PEAK POINT
= MOLTEN STEEL

TO FIRST
DETERMINATION STEP ST5

TO SECOND
DETERMINATION STEP ST6

FIG. 4A

BACKGROUND  MOLTEN STEEL  BACKGROUND
FLOW F

FIG. 4B

FIG. 5A

BACKGROUND    MOLTEN STEEL    BACKGROUND
                  FLOW F

PIXEL REGION DETERMINED
TO CORRESPOND TO SLAG S

FIG. 5B

FIG. 6

FIG. 7

FIG. 8

## FIG. 9A

BACKGROUND  MOLTEN STEEL  BACKGROUND
FLOW F

PIXEL REGION DETERMINED
TO CORRESPOND TO SLAG S

## FIG. 9B

## FIG. 10A

## FIG. 10B

BACKGROUND  MOLTEN STEEL  BACKGROUND
FLOW F

PIXEL REGION DETERMINED
TO CORRESPOND TO SLAG S

**EP 3 584 329 A1**

## INTERNATIONAL SEARCH REPORT

International application No.

PCT/JP2018/004818

| A. CLASSIFICATION OF SUBJECT MATTER |
|---|
| Int.Cl. C21C5/46(2006.01)i, G01N21/17(2006.01)i, G01N33/20(2006.01)i |

According to International Patent Classification (IPC) or to both national classification and IPC

| B. FIELDS SEARCHED |
|---|
| Minimum documentation searched (classification system followed by classification symbols)<br>Int.Cl. C21C5/46, G01N21/17, G01N33/20 |

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2010-111925 A (JFE STEEL CORPORATION) 20 May 2010, entire text, fig. 1-6 (Family: none) | 1–5 |
| A | JP 2007-246959 A (NIPPON STEEL CORPORATION) 27 September 2007, entire text, fig. 1-11 (Family: none) | 1–5 |
| A | JP 2003-19553 A (NIPPON KOKAN KK) 21 January 2003, entire text, fig. 1-6 (Family: none) | 1–5 |

☐ Further documents are listed in the continuation of Box C.　　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>29.03.2018 | Date of mailing of the international search report<br>10.04.2018 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017025441 A
- JP 2001107127 A

- JP 2006213965 A